Europäisches Patentamt

European Patent Office

Office européen des brevets

(19)

(11) Publication number: **0 439 115 B1**

(12) **EUROPEAN PATENT SPECIFICATION**

(45) Date of publication of patent specification: **18.10.95**

(51) Int. Cl.6: **C07C 45/41**, C07C 47/02, C07C 47/32, B01J 23/26, B01J 35/10

(21) Application number: **91100749.0**

(22) Date of filing: **22.01.91**

The file contains technical information submitted after the application was filed and not included in this specification

(54) **Process for producing aliphatic or alicyclic aldehydes.**

(30) Priority: **22.01.90 JP 12233/90**

(43) Date of publication of application:
**31.07.91 Bulletin 91/31**

(45) Publication of the grant of the patent:
**18.10.95 Bulletin 95/42**

(84) Designated Contracting States:
**CH DE FR GB LI**

(56) References cited:
**EP-A- 0 150 961**

**PATENT ABSTRACTS OF JAPAN, vol. 11, no. 192 (C-429)[2639], 19th June 1987; & JP-A-62 14 942**

**PATENT ABSTRACTS OF JAPAN, vol. 9, no. 317 (C-319)[2040], 12th December 1985; & JP-A-60 152 434**

**PATENT ABSTRACTS OF JAPAN, vol. 11, no. 322 (C-453)[2769], 20th October 1987; & JP-A-62 108 832**

(73) Proprietor: **Mitsubishi Chemical Corporation**
**5-2, Marunouchi 2-chome**
**Chiyoda-ku**
**Tokyo (JP)**

(72) Inventor: **Yokoyama, Toshiharu**
**3-3, Tsutsujigaoka,**
**Midori-ku**
**Yokohama-shi,**
**Kanagawa (JP)**
Inventor: **Matsuyama, Naoko**
**15-12-A201, Sumiyoshidai,**
**Midori-ku**
**Yokohama-shi,**
**Kanagawa (JP)**
Inventor: **Maki, Takao**
**11-6, Kugenumakaigan 7-chome**
**Fujisawa-shi,**
**Kanagawa (JP)**

(74) Representative: **VOSSIUS & PARTNER**
**Postfach 86 07 67**
**D-81634 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a process for producing aliphatic or alicyclic aldehydes which are useful as an intermediate in organic syntheses.

While various processes for producing aliphatic aldehydes have been proposed to date, an optimal means for starting with carboxylic acids or derivatives thereof have not yet been reported.

The most commonly employed of the conventional processes is a process utilizing so-called Rosenmund reduction of a carboxylic acid chloride. This process has the disadvantage of high cost incurred.

Direct reduction of carboxylic acids with molecular hydrogen would be the most advantageous process for the production of aldehydes, but it has been regarded extremely difficult.

As the latest technique, U.S. Patent 4,328,373 proposes a process in which methyl isobutyrate or methyl pivalate is hydrogenated in a gaseous phase in the presence of a yttrium oxide catalyst to obtain a corresponding aldehyde. This process, however, starts with a methyl ester of a carboxylic acid, but not with a free carboxylic acid. Moreover, the temperature employed is high throughout the reaction, and the yield of the desired aldehyde is low due to side reactions.

A process in which an aliphatic carboxylic acid is hydrogenated in the presence of zirconium oxide as a catalyst to obtain a corresponding aldehyde is disclosed in JP-A-62-108832 (the term "JP-A" as used herein means an "unexamined published Japanese patent application"). As a result of further investigations, however, it turned out that a zirconium oxide catalyst which is prepared by conventional processes needs further improvements to be used in the above-described process, for example, in activity, yield of desired products, working life, etc. In particular, when the starting aliphatic carboxylic acid has a large number of carbon atoms, the yield attained is low.

In EP-A-150 961 there is disclosed a process for the production of aromatic aldehydes by catalytic hydrogenation of aromatic carboxylic acids or esters thereof with a zirconium oxide catalyst which can contain chromium. The catalyst has a volume of pores having radii not greater than 100 Å of at least 0.05 ml/g.

An object of the present invention is to provide a process for producing aliphatic or alicyclic aldehydes in high yield directly from a corresponding aliphatic or alicyclic carboxylic acid or a derivative thereof.

The present invention relates to a process for producing an aliphatic or alicyclic aldehyde comprising the step of hydrogenating an aliphatic or alicyclic carboxylic acid or a derivative thereof with molecular hydrogen in the presence of a catalyst, wherein the catalyst is a zirconium oxide catalyst which contains chromium as an essential component, has a weakly basic site amount of more than 0.03 mmol/g as determined by a temperature programmed desorption method using carbon dioxide as an adsorbate in which the amount of carbon dioxide desorbed in the temperature range of from 100 to 250°C is measured, and has pores having a radius of from 20 to 500 Å in an amount of not less than 0.1 $cm^3$/g and pores having a radius of from 1,000 to 50,000 Å in an amount of not less than 0.05 $cm^3$/g as measured with a mercury porosimeter.

It is known that acid-base characteristics of zirconium oxide solid catalysts containing chromium as an essential component are subject to wide variations depending on impurity contents in raw materials and methods of preparation.

Various means for determining acid-base characteristics of a solid are known. Inter alia, an indicator adsorption method (Yuki Gosei Kagaku Kyokai-shi (Journal of The Society of Synthetic Organic Chemistry, Japan), Vol. 33, No. 11, p. 842 (1975)) is commonly used. However, this method is unsuitable for determining colored substances such as the catalyst used in the present invention.

On the other hand, there is a method for examining acid-base characteristics of a solid catalyst in which a basic gas, e.g., ammonia, or an acidic gas, e.g., carbon dioxide, is once adsorbed on the surface of a solid, then desorbing the adsorbed gas by increasing a temperature at a given rate, and the amount of desorbed gas is measured, known as a temperature programmed desorption method (Shokubai Koza (Lecture on Catalyst), Vol. 3, pp. 145-156, published by Kodansha, Japan on 1985.

According to this method, characteristics of basic sites or acidic sites on the catalyst surface can be determined by using carbon dioxide or ammonia gas as an adsorbate molecule, respectively. That is, the temperature at which the gas is desorbed shows strength, and the amount of desorbed gas shows the amount of acidic sites or basic sites.

Various zirconium oxide catalysts essentially containing chromium have been examined according to the above-mentioned temperature programmed desorption method and, as a result, it has been found that the amount of weakly basic sites on the catalyst surface (hereinafter referred to as "weakly basic site amount") relates to the activity of the catalyst. Namely, it was elucidated that, when the amount of carbon dioxide, desorbed in the temperature range of from 100 to 250°C in a temperature programmed desorption method using carbon dioxide, is being taken as the weakly basic site amount, a catalyst having a weakly basic site amount of more than 0.03 mmol/g is particularly excellent in catalyzing activity.

In determining weakly basic sites on the surface of a solid catalyst by a temperature programmed desorption method, the absolute adsorption and desorption amounts of carbon dioxide vary depending on the apparatus or operation used. On this account, for reference, a weakly basic site amount of standard active alumina ALO-4 specified by Catalysis Society of Japan has been determined under the same conditions for a temperature programmed method. As a result, ALO-4 was found to have a weakly basic site amount of 0.03 mmol/g or less. Accordingly, the catalyst which can be used in the present invention is characterized by having a greater weakly basic site amount than ALO-4.

The carbon dioxide desorbed and analyzed in a low temperature range of from 100 to 250°C had been adsorbed at weakly basic sites on the catalyst surface.

If the weakly basic site amount is 0.03 mmol/g or less, the reaction results of hydrogenation particularly of aliphatic carboxylic acids are considerably deteriorated.

It appears that various factors account for the basic characteristics of the catalyst as above determined. In particular, the presence of impurities has a great influence. For example, where the zirconium oxide catalyst is prepared by refining raw ore, impurities such as sulfur and halogens sometimes remain depending on the process employed. These impurities cause wide variations of acid-base characteristics of the surface of the resulting catalyst. The same is applies to chromium raw materials used as an essential component.

Therefore, in the preparation of the catalyst to be used in the present invention, it is required to purify the raw materials used for the zirconium oxide catalyst and the chromium compounds.

Raw materials for the zirconium oxide catalyst which can be used in the present invention include commercially available zirconium hydroxide (e.g., zirconyl hydroxide, zirconium hydroxide) and zirconyl carbonate. Commercially available zirconium oxide is also employable as a raw material.

An impurity-free zirconium oxide catalyst can be obtained by firing granules of the raw material, e.g., commercially available zirconium oxide, zirconyl hydroxide, zirconium hydroxide and zirconyl carbonate. Use of commercially available zirconium oxide is not recommended as the raw material because it may contain co-fired impurities on its surface which are difficult to be removed. Therefore, a zirconium oxide catalyst prepared by firing the raw material, such as zirconium hydroxide, zirconyl hydroxide or zirconyl carbonate, at a temperature of from about 300 to 950°C prior to molding is preferred. More preferably, firing of these raw materials is conducted after molding. In raw materials which are used for the preparation of a zirconium oxide catalyst, the amounts of impurities, e.g., sulfur and halogens, more or less differ depending on the process for preparing the raw material itself. A zirconium oxide catalyst which has been prepared from the raw materials containing large amounts of impurities is not suitable in the present invention.

It is preferred that, if desired, impurity contents of raw materials can be reduced before molding or firing by known methods, such as washing with water or diluted aqueous ammonia.

It is particularly necessary to reduce the sulfur content in the raw material to 0.1% by weight or less because sulfur is present as a sulfuric acid ion and seriously reduces the weakly basic site amount on the catalyst surface.

Chromium compounds which can be used in the preparation of the catalyst include inorganic chromium compounds, e.g., chromium sulfate, chromium nitrate, chromium halides, and chromium (VI) oxide, bichromic acid or an ammonium or alkali metal salt thereof; and organic chromium salts, e.g., chromium formate, chromium acetate, and chromium oxalate. Because they contain no general catalyst poisons, preferred chromium compounds are those which decompose at relatively low temperatures and contain no poisoning elements, such as chromium nitrate, chromium anhydride salts, ammonium bichromate, chromium acetate, chromium formate, and chromium oxalate.

The atomic ratio of chromium to zirconium (Cr/Zr) in the catalyst of the present invention ranges from about 0.001 to about 0.5, and preferably from 0.01 to 0.3.

In the process of the present invention, catalytic activity is closely related to the volume and distribution of pores. It is required for the catalyst to have not less than 0.1 cm$^3$/g of pores having a radius of from 20 to 500 Å and not less than 0.05 cm$^3$/g of pores having a radius of from 1,000 to 50,000 Å.

If the volume of pores having a radius of from 1,000 to 50,000 Å is less than 0.05 cm$^3$/g, diffusion of the reactant and reaction product in the pores is considerably retarded even if other requirements, i.e., weakly basic site amount and volume of pores having a radius of from 20 to 500 Å, are satisfied. Such being the case, the reaction activity is reduced, and the product undergoes successive reactions, resulting in a reduction in aldehyde selectivity.

The catalyst according to the present invention can be prepared in a conventional manner. For example, a chromium salt aqueous solution and zirconyl hydroxide powder are mixed, if desired, in the presence of an appropriate binder, and the mixture is extrusion-molded, dried, and fired at a prescribed temperature. This process being followed, a pore distribution and a pore volume can be appropriately adjusted by dehydration and decomposition of raw materials during drying and firing and, if desired, removal of organic binders added (e.g., polyvinyl alcohol, starch paste, crystalline cellulose, various surface active agents, and low-melting waxes) by combustion.

It should be noted that the tablet compressing method, which is a commonly employed molding technique for solid catalysts, is not favorable in the present invention because it causes destruction of pores having a radius of from 1,000 to 50,000 Å. It is a matter of course that the tablet compressing method can be employed as far as the volume of pores having a radius of from 1,000 to 50,000 Å may be maintained at 0.05 $cm^3$/g or more by using an organic binder removable by combustion.

Binders which may be added if desired preferably include organic compounds which disappear on firing. Inorganic binders tend to remain on the catalyst surface after firing, adversely affecting reaction activity. Preferred organic binders include those generally employed for granulation, such as polyvinyl alcohol, starch paste, crystalline cellulose, surface active agents, low-melting waxes, and stearic acid.

A preferred temperature for firing granules ranges from 400 to 1,100°C, and more preferably from 400 to 900°C.

Carboxylic acids which can be used as a starting material in the process of the present invention include aliphatic carboxylic acids, alicyclic carboxylic acids, and derivatives of these carboxylic acids. Preferred derivatives include esters and anhydrides.

Specific examples of the aliphatic carboxylic acids include straight chain or branched and saturated or unsaturated carboxylic acids having from 4 to 24 carbon atoms, such as butyric acid, isobutyric acid, pivalic acid, valeric acid, hexanoic acid, heptanoic acid, octanoic acid, 2-ethylhexanoic acid, nonanoic acid, decanoic acid, undecanoic acid, lauric acid, tridecanoic acid, tetradecanoic acid, pentadecanoic acid, hexadecanoic acid, heptadecanoic acid, stearic acid, isostearic acid, nonadecanoic acid, eicosanoic acid, heneicosanoic acid, docosanoic acid, tricosanoic acid, tetracosanoic acid, 10-undecenoic acid, oleic acid, and 11-eicosenoic acid.

Specific examples of the alicyclic carboxylic acids include cyclopentanecarboxylic acid and cyclohexanecarboxylic acid.

These aliphatic or alicyclic carboxylic acids may be substituted with reaction inert groups, such as an aryl group and an alkoxy group.

Carboxylic acid derivatives which can be used as a starting compound include esters, such as methyl, ethyl, n-butyl, cyclohexyl, and phenyl esters, and anhydrides, such as homoanhydride and heteroanhydrides. Specific examples of these derivatives include methyl laurate, n-butyl laurate, methyl stearate, n-butyl stearate, lauric anhydride, and stearic anhydride.

In the present invention, a carboxylic acid containing from 4 to 24 carbon atoms or a derivative thereof is generally used as a starting compound. Considering that the catalyst to be used has pores of from 1,000 to 50,000 Å in radius and that such a pore size distribution is related to diffusion of the starting material and the reaction product as stated above, the present invention exhibits particularly pronounced effects when applied to compounds having a relatively large-sized molecule, i.e., higher aliphatic carboxylic acids having from about 8 to about 22, and particularly from 12 to 22, carbon atoms.

Hydrogenation is advantageously carried out in the gaseous phase. The reaction temperature is generally from 200 to 500°C, and preferably from 250 to 400°C. The reaction pressure is generally normal pressure or, if desired, slighty increased pressure.

Where the catalyst is used as a fixed bed, the starting carboxylic acid or its derivative is generally fed at a space velocity ranging from about 0.01 to about 1 $hr^{-1}$, and preferably from 0.03 to 0.5 $hr^{-1}$, in terms of LHSV (liquid hourly space velocity).

The space velocity of hydrogen is generally in the range of from about 100 to about 20,000 $hr^{-1}$, and preferably from 500 to 5,000 $hr^{-1}$, in terms of GHSV (gas hourly space velocity). Hydrogen to be fed may contain small amounts of inert gases such as nitrogen, water vapor, and carbon dioxide.

The present invention is now illustrated in greater detail by way of the following Examples and Comparative Examples, but it should be understood that the present invention is not deemed to be limited thereto. All the percents, parts, and ratios are by weight unless otherwise indicated.

EXAMPLE 1 AND COMPARATIVE EXAMPLES 1 AND 2

Preparation of Catalysts:

Catalyst A (Example 1):

Commercially available zirconyl hydroxide was thoroughly washed with diluted aqueous ammonia and water to remove impurities and dried. To 2,340 g of the thus treated zirconyl hydroxide ($ZrO(OH)_2$, $ZrO_2$ contents: 85.4%) was added an aqueous solution consisting of 325 g of chromium nitrate nonahydrate, 50 g of polyvinyl alcohol ("EG05" produced by Nippon Gosei K.K.), and 940 g of water, and the mixture was kneaded in a kneader and extruded by means of an extrusion molding machine to obtain a strand having a diameter of 3 mm. The extruded strand was cut to a length of about 5 mm, dried, and fired at 700°C for 3 hours to obtain a catalyst (designated catalyst A). The sulfur content of catalyst A was found to be 0.02%.

Catalyst B (Comparative Example 1):

Catalyst B was prepared in the same manner as for catalyst A, except for using commercially available zirconyl hydroxide as such without removing impurities. Catalyst B had a sulfur content of 0.2%. Catalyst C (Comparative Example 2):

The same raw materials at the same mixing ratio as used for catalyst A were kneaded, dried, and fired at 600°C for 3 hours. One part of stearic acid was mixed with 100 parts of the fired product, and the mixture was formed into tablets having a diameter of 5 mm and a length of 5 mm by the tablet compressing method. The tablets were fired at 700°C for 3 hours to prepare catalyst C.

Determination of Physical Properties of Catalysts:

1) Surface Basicity:

Surface basicity of catalysts A, B and C was determined by a temperature programmed desorption method using carbon dioxide as an adsorbate. The determination was made in accordance with a standard method described in Shokubai Jikken Handbook (Catalyst Experimental Handbook), separate volume of Shokubai Koza (Lectures on Catalyst), p. 175, published by Kodansha, Japan on 1986 as follows.

The catalyst was ground to 10 to 20 mesh, and a 20 mg portion was placed in a U-tube made of quartz (inner diameter: 3 mm). The U-tube was set in an apparatus, the atmosphere was displaced with helium gas, and the catalyst was heated at 600°C for 1 hour. The catalyst was then reduced with hydrogen at 350°C for 1 hour while feeding 50 mℓ/min of $H_2$ gas. The temperature was lowered to 100°C, and the tube was purged with helium gas. Pulses (1 mℓ) of helium gas containing 10% carbon dioxide were introduced at 100°C until adsorption saturation was reached, followed by purging with helium gas. The temperature was raised up to 600°C at a rate of 10°C/min while feeding 40 mℓ/min of helium gas, and desorbed gas was analyzed by means of a thermal conductivity cell to obtain a chromatogram. The above-described operation was repeated, except that carbon dioxide was not adsorbed to obtain a base line which should be subtracted from the resulting chromatogram.

For reference, surface basicity of high-purity active alumina, ALO-4, specified by Catalysis Society of Japan (specific surface area: 177 m²/g) was also determined in the same manner as described above.

2) Pore Volume (mℓ/g):

Measured with a mercury porosimeter.

3) Specific Surface Area:

Measured in accordance with a BET method.

The results of these physical properties determinations are shown in Table 1 below. As shown in Table 1, catalysts A, B and C had substantially the same specific surface area (about 70 m²/g) but differed in surface base site amount and pore structure.

Hydrogenation of Lauric Acid:

Hydrogenation of lauric acid was carried out at a varied reaction temperature using a given amount of catalyst A, B or C under the following conditions.

Pressure: normal pressure
Space Velocity of Acid (LHSV): 0.14 hr⁻¹
Space Velocity of Hydrogen (GHSV): 1,250 hr⁻¹

The yield of lauric aldehyde was obtained by multiplying (conversion of lauric acid) by (selectivity of lauric aldehyde). The conversion of lauric acid (%), selectivity of lauric aldehyde (%), and space time yield of lauric aldehyde (mol/kg-catalyst・hr) obtained under the temperature condition which gave the maximum yield as obtained above are shown in Table 1.

## TABLE 1

| Catalyst | | CO$_2$ Desorption at 100-250°C (mmol/g-catalyst) | Pore Volume (ml/g) | | Specific Surface Area (m$^2$/g) | Reaction Temperature (°C) | Conversion of Lauric Acid (%) | Selectivity of Lauric Aldehyde (%) | Space Time Yield of Lauric Aldehyde |
|---|---|---|---|---|---|---|---|---|---|
| | | | 20-500Å | 1,000-50,000Å | | | | | |
| Example 1 | A | 0.080 | 0.18 | 0.19 | 72 | 325 | 89 | 85.2 | 0.525 |
| Comparative Example 1 | B | 0.014 | 0.18 | 0.18 | 69 | 360 | 92.8 | 77.6 | 0.423 |
| Comparative Example 2 | C | 0.080 | 0.17 | 0.04 | 77 | 350 | 80.5 | 63.8 | 0.331 |
| Reference | Al$_2$O$_3$ | 0.0285 | — | — | 177 | — | — | — | — |

## EXAMPLE 2

Hydrogenation of stearic acid was conducted under the following conditions using catalyst A as prepared in Example 1.

Pressure: normal pressure

Temperature: 315°C

Space Velocity of Acid (LHSV): 0.11 hr$^{-1}$

Space Velocity of Hydrogen (GHSV): 1,250 hr$^{-1}$

As a result, the conversion of stearic acid was 91.7%, the selectivity of stearic aldehyde was 84.6%, the space time yield of stearic aldehyde was 0.33 mol/kg-catalyst·hr.

## EXAMPLE 3

Hydrogenation of n-octanoic acid was conducted under the following conditions using catalyst A as prepared in Example 1.

Pressure: normal pressure

Temperature: 330°C

Space Velocity of Acid (LHSV): 0.11 hr$^{-1}$

Space Velocity of Hydrogen (GHSV): 1,250 hr$^{-1}$

As a result, the conversion of n-octanoic acid was 96.9%, the selectivity of n-octanal was 72.9%, and the space time yield of n-octanal was 0.57 mol/kg-catalyst·hr.

## EXAMPLE 4

Hydrogenation of pivalic acid was conducted under the following conditions using catalyst A as prepared in Example 1.

Pressure: normal pressure

Temperature: 340°C

Space Velocity of Acid (LHSV): 0.13 Hr$^{-1}$

Space Velocity of Hydrogen (GHSV): 400 hr$^{-1}$

As a result, the conversion of pivalic acid was 97.0%, the selectivity of pivalic aldehyde was 99.5%, and the space time yield of pivalic aldehyde was 1.1 mol/kg-catalyst·hr.

## EXAMPLE 5

Hydrogenation of cyclohexanecarboxylic acid was conducted under the following conditions using catalyst A as prepared in Example 1.

Pressure: normal pressure

Temperature: 330°C

Space Velocity of Acid (LHSV): 0.097 hr$^{-1}$

Space Velocity of Hydrogen (GHSV): 1250 hr$^{-1}$

As a result, the conversion of cyclohexanecarboxylic acid was 97.1%, the selectivity of cyclohexanecarbaldehyde was 98.9%, and the space time yield of cyclohexanecarbaldehyde was 0.70 mol/kg-catalyst·hr.

EXAMPLE 6

Hydrogenation of methyl stearate was carried out under the following conditions using catalyst A as prepared in Example 1.

Pressure: normal pressure
Temperature: 310°C
Space Velocity of Ester (LHSV): 0.12 hr$^{-1}$
Space Velocity of Hydrogen (GHSV): 1250 hr$^{-1}$

As a result, the conversion of methyl stearate was 83.0%, the selectivity of stearic aldehyde was 72.6%, and the space time yield of stearic aldehyde was 0.23 mol/kg-catalyst•hr.

COMPARATIVE EXAMPLE 3

Hydrogenation of cyclohexane carboxylic acid was conducted in the same manner as in Example 5, escept for using catalyst B and at the temperature of 350°C.

As a result, the conversion of cyclohexane carboxylic acid was 92.9%, the selectivity of cyclohexanecarbaldehyde was 96.4%, and the space time yield of cyclohexanecarbaldehyde was 0.60 mol/kg-catalyst•hr.

As described in the foregoing, an aliphatic or alicyclic aldehyde can be produced in high yield directly from a corresponding aliphatic or alicyclic carboxylic acid or a derivative thereof by the process according to the present invention.

**Claims**

1. A process for producing aliphatic or alicyclic aldehydes comprising the step of hydrogenating an aliphatic or alicyclic carboxylic acid or a derivative thereof with molecular hydrogen in the presence of a catalyst, characterized in that the

   catalyst is a zirconium oxide catalyst which contains chromium as an essential component; has a weakly basic site amount of more than 0.03 mmol/g as determined by a temperature programmed desorption method using carbon dioxide as an adsorbate in which the amount of carbon dioxide desorbed in the temperature range of from 100 to 250°C is measured; and has pores having a radius of from 20 to 500 Å in an amount of not less than 0.1 cm$^3$/g and pores having a radius of from 1,000 to 50,000 Å in an amount of not less than 0.05 cm$^3$/g as measured with a mercury porosimeter.

2. A process as claimed in claim 1, wherein said zirconium oxide catalyst is the one prepared by the steps of: mixing a raw material for zirconium oxide, a chromium compound, and an organic binder removable on combustion;

extrusion-molding the mixture; drying; and firing at 400 to 1,100°C.

3. A process as claimed in claim 2, wherein said raw material for zirconium oxide catalyst is zirconyl hydroxide, zirconium hydroxide, zirconyl carbonate, and/or commercially available zirconium oxide.

4. A process as claimed in claim 2, wherein said chromium compound is chromium sulfate, chromium nitrate, a chromium halide, chromium (VI) oxide, bichromic acid or an ammonium or alkali metal salt thereof, chromium formate, chromium acetate, and/or chromium oxalate.

5. A process as claimed in claim 2, wherein said raw material for zirconium oxide catalyst and chromium compound are mixed at a Cr/Zr atomic ratio of from 0.001 to 0.5.

6. A process as claimed in claim 1, wherein said aliphatic or alicyclic carboxylic acid or a derivative thereof is an aliphatic or alicyclic carboxylic acid which may have a reaction inert substituent, or an ester or anhydride thereof.

7. A process as claimed in claim 6, wherein said aliphatic carboxylic acid is a saturated or unsaturated carboxylic acid having from 4 to 24 carbon atoms.

8. A process as claimed in claim 7, wherein said alicyclic caboxylic acid is cyclohexanecarboxylic acid.

9. A process as claimed in claim 1, wherein said hydrogenating is carried out at a temperature of from 200 to 500°C.

10. A process as claimed in claim 1, wherein said catalyst is a fixed bed catalyst, said aliphatic or alicyclic carboxylic acid or a derivative thereof is fed at a liquid hourly space velocity of from 0.01 to 1 hr$^{-1}$, and hydrogen is fed at a gas hourly space velocity of from 100 to 20,000 hr$^{-1}$.

**Patentansprüche**

1. Verfahren zur Herstellung von aliphatischen oder alicyclischen Aldehyden, umfassend die Stufe der Hydrierung einer aliphatischen oder alicyclischen Carbonsäure oder eines Derivates davon mit molekularem Wasserstoff in Gegenwart eines Katalysators, dadurch gekennzeichnet, daß der Katalysator ein Zirconiumoxid-Katalysator ist, der Chrom als wesentlichen Bestandteil enthält, eine Menge von schwach basischen Stellen von mehr als 0,03 mmol/g besitzt, wie durch eine Temperatur-programmierte Desorptionsmethode unter Verwendung von Kohlendioxid als Adsorbat bestimmt wird, bei der die Menge Kohlendioxid gemessen wird, die im Temperaturbereich von 100 bis 250 °C desorbiert wird, und Poren mit einem Radius von 20 bis 500 Å in einer Menge von nicht weniger als 0,1 cm$^3$/g und Poren mit einem Radius von 1 000 bis 50 000 Å in einer Menge von nicht weniger als 0,05 cm$^3$/g, wenn mit einem Quecksilber-Porosimeter gemessen, besitzt.

2. Verfahren nach Anspruch 1, worin der genannte Zirconiumoxid-Katalysator ein solcher ist, der durch die Stufen des Mischens eines Rohmaterials von Zirconiumoxid, einer Chromverbindung und einem organischen, durch Verbrennung entfernbaren Bindemittels; Formung der Mischung durch Extrudieren; Trocknung und Verglühen bei 400 bis 1 100 °C hergestellt wurde.

3. Verfahren nach Anspruch 2, worin das genannte Rohmaterial für den Zirconiumoxid-Katalysator Zirconylhydroxid, Zirconiumhydroxid, Zirconylcarbonat und/oder im Handel erhältliches Zirconiumoxid ist.

4. Verfahren nach Anspruch 2, worin die genannte Chromverbindung Chromsulfat, Chromnitrat, ein Chromhalogenid, Chrom(VI)-oxid, Dichromsäure oder ein Ammonium- oder Alkalimetallsalz davon, Chromformat, Chromacetat und/oder Chromoxalat ist.

5. Verfahren nach Anspruch 2, worin das genannte Rohmaterial für den Zirconiumoxid-Katalysator und die Chromverbindung in einem Cr/Zr-Atomverhältnis von 0,001 bis 0,5 gemischt werden.

6. Verfahren nach Anspruch 1, worin die genannte aliphatische oder alicyclische Carbonsäure oder ein Derivat davon eine aliphatische oder alicyclische Carbonsäure, die einen reaktionsinerten Substituenten besitzen kann, oder ein Ester oder Anhydrid davon ist.

7. Verfahren nach Anspruch 6, worin die genannte aliphatische Carbonsäure eine gesättigte oder ungesättigte Carbonsäure mit 4 bis 24 Kohlenstoffatomen ist.

8. Verfahren nach Anspruch 7, worin die genannte aliphatische Carbonsäure Cyclohexancarbonsäure ist.

9. Verfahren nach Anspruch 1, worin die genannte Hydrierung bei einer Temperatur von 200 bis 500 °C durchgeführt wird.

10. Verfahren nach Anspruch 1, worin der genannte Katalysator ein Festbett-Katalysator ist, die genannte aliphatische oder alicyclische Carbonsäure oder ein Derivat davon mit einer stündlichen Flüssigkeits-Raumgeschwindigkeit von 0,01 bis 1 h$^{-1}$ und Wasserstoff mit einer stündlichen Gas-Raumgeschwindigkeit von 100 bis 20 000 h$^{-1}$ zugeführt wird.

**Revendications**

1. Procédé pour la production d'aldéhydes aliphatiques ou alicycliques comprenant l'étape d'hydrogénation d'un acide carboxylique aliphatique ou alicyclique ou d'un de ses dérivés à l'aide de l'hydrogène moléculaire en présence d'un catalyseur, caractérisé en ce que

le catalyseur est un catalyseur oxyde de zirconium qui contient, comme composant essentiel, du chrome ; il a une quantité de sites faiblement basiques, déterminée par une méthode de désorption programmée en température en utilisant le dioxyde de carbone comme adsorbat et dans laquelle on mesure la quantité de dioxyde de carbone désorbé dans l'intervalle des températures allant de 100 à 250 °C, supérieure à 0,03 mmole/g ; il a une quantité de pores dont le rayon est situé entre 20 et 500 Å qui n'est pas inférieure à 0,1 cm$^3$/g et une quantité de pores dont le rayon est situé entre 1 000 et 50 000 Å qui n'est pas inférieure à 0,05 cm$^3$/g, quantités mesurées par un porosimètre à mercure.

2. Procédé selon la revendication 1, dans lequel ledit catalyseur oxyde de zirconium est celui préparé par les étapes de : mélange d'une matière première pour l'oxyde de zirconium, d'un composé du chrome, et d'un liant organique pouvant s'éliminer par combustion ; moulage du mélange par extrusion ; séchage ; et cuisson entre 400 et 1 100 °C.

3. Procédé selon la revendication 2, dans lequel ladite matière première pour le catalyseur oxyde de zirconium est de l'hydroxyde de zirconyle, de l'hydroxyde de zirconium, du carbonate de zirconyle, et/ou de l'oxyde de zirconium commercial.

4. Procédé selon la revendication 2, dans lequel ledit composé du chrome est du sulfate de chrome, du nitrate de chrome, un halogénure de chrome, de l'oxyde de chrome (VI), de l'acide bichromique ou un de ses sels d'ammonium ou de métal alcalin, du formiate de chrome, de l'acétate de chrome, et/ou de l'oxalate de chrome.

5. Procédé selon la revendication 2, dans lequel ladite matière première pour le catalyseur oxyde de zirconium et le composé du chrome sont mélangés à un rapport atomique Cr/Zr situé entre 0,001 et 0,5.

6. Procédé selon la revendication 1, dans lequel ledit acide carboxylique aliphatique ou alicyclique ou un de ses dérivés est un acide carboxylique aliphatique ou alicyclique qui peut avoir un substituant réactionnellement inerte, ou un de ses esters ou anhydrides.

7. Procédé selon la revendication 6, dans lequel ledit acide carboxylique aliphatique est un acide carboxylique saturé ou insaturé ayant de 4 à 24 atomes de carbone.

8. Procédé selon la revendication 7, dans lequel ledit acide carboxylique alicyclique est l'acide cyclohexanecarboxylique.

9. Procédé selon la revendication 1, dans lequel ladite hydrogénation est conduite à une température de 200 à 500°C.

10. Procédé selon la revendication 1, dans lequel ledit catalyseur est un catalyseur en lit fixe, ledit acide carboxylique aliphatique ou alicyclique ou un de ses dérivés est admis à une vitesse spatiale horaire de liquide de 0,01 à 1 $h^{-1}$, et l'hydrogène est admis à une vitesse spatiale horaire de gaz de 100 à 20 000 $h^{-1}$.